(19) Europäisches Patentamt
European Patent Office
Office européen des brevets

(11) Veröffentlichungsnummer: **0 006 990**
**A1**

(12) # EUROPÄISCHE PATENTANMELDUNG

(21) Anmeldenummer: 79101618.1

(22) Anmeldetag: 28.05.79

(51) Int. Cl.³: C 07 C 155/02
C 07 C 125/06

(30) Priorität: 14.06.78 DE 2826012

(43) Veröffentlichungstag der Anmeldung: 23.01.80 Patentblatt 80/2

(84) Benannte Vertragsstaaten: BE CH DE FR GB

(71) Anmelder: BASF Aktiengesellschaft
Carl-Bosch-Strasse 38
D-6700 Ludwigshafen(DE)

(72) Erfinder: Loeffler, Hans-Peter, Dr.
Defreggerstrasse 14
D-6700 Ludwigshafen(DE)

(72) Erfinder: Thym, Sabine, Dr.
Hasenhain 20
D-6900 Heidelberg-Dossenheim(DE)

(72) Erfinder: Koenig, Karl-Heinz, Dr.
Pierstrasse 8 a
D-6710 Frankenthal(DE)

(72) Erfinder: Zeeh, Bernd, Dr.
Thorwaldsenstrasse 5
D-6700 Ludwigshafen(DE)

(54) **Neue N,N-Bis-halogenmethyl-carbamidsäureester und Verfahren zu ihrer Herstellung.**

(57) Neue N,N-Bis-halogenmethyl- carbamidsäureester und Verfahren zu ihrer Herstellung durch Umsetzung von N,N-Bis-halogenmethyl- carbamidsäurehalogeniden mit Mercaptanen oder Hydroxylverbindungen und basischen Verbindungen oder mit Salzen der Mercaptane oder Hydroxyverbindungen.

Die nach dem Verfahren der Erfindung hergestellten neuen N,N-Bis-halogenmethyl- carbamidsäureester I sind wertvolle Ausgangsstoffe für die Herstellung von Farbstoffen, Pflanzenschutzmitteln, Textilausrüstungsmitteln zur Hochveredelung.



Croydon Printing Company Ltd

BASF Aktiengesellschaft O.Z. 0050/033225

## Neue Bis-(N-Halogenmethyl)-carbamidsäureester und Verfahren zu ihrer Herstellung

Die Erfindung betrifft neue Bis-(N-Halogenmethyl)-carbamidsäureester und Verfahren zu ihrer Herstellung durch Umsetzung von Bis-(N-Halogenmethyl)-carbamidsäurehalogeniden mit Mercaptanen oder Hydroxylverbindungen und basischen Verbindungen oder mit Salzen der Mercaptane oder Hydroxylverbindungen.

Es ist aus der deutschen Offenlegungsschrift 17 68 983 bekannt, daß man Urethane bei -10 bis +150°C mit mindestens 2 Mol Formaldehyd in Gegenwart von Natronlauge umsetzt und dann das Gemisch mit mindestens stöchiometrischen Mengen eines Halogenierungsmittels umsetzt. Die Offenlegungsschrift lehrt, daß sich Bis-(N-Halogenmethyl)-carbamidsäureester nicht dadurch herstellen lassen, daß man Bis-(N-Chlormethyl)-carbamidsäurechloride mit Alkoholen umsetzt, und daß in einem solchen Falle die Halogenatome der α-Halogenmethylgruppe mit Alkohol oder Alkoholaten reagieren, das Chloratom des Carbamoylchlorid-Restes aber nicht substituiert wird. Als Umsetzungsprodukt erhält man nach der Lehre dieser Offenlegungsschrift Bis-(N-Alkoxymethyl)-carbamidsäurechloride:

$$(ClCH_2)_2N\overset{O}{\overset{\|}{C}}Cl + ROH \longrightarrow (ROCH_2)_2N\overset{O}{\overset{\|}{C}}Cl \ .$$

Es wird in diesem Zusammenhang auf die belgische Patentschrift 660 727 verwiesen. Die Beispiele dieser belgischen Patentschrift zeigen im wesentlichen die Umsetzung von Monohalogenmethyl-carbamidsäurehalogeniden und heterocyclischen Carbamidsäurehalogeniden, deren Stickstoffatom ein Glied des heterocyclischen Ringes bildet, mit Alkoholen, in der Hauptsache Alkanolen, im alkalischen und im saueren Medium. Bis-(N-Halogenmethyl)-carbamidsäurehalogenide werden eher im saueren Medium (Beispiele 9, 40 und 49) umgesetzt, lediglich in 2 Beispielen (Beispiel 2 und 3) werden sie nur im Falle von Methanol mit Natrium- oder Magnesiummethylat umgesetzt.

Es wurde nun gefunden, daß man Bis-(N-Halogenmethyl)-carbamidsäureester der Formel

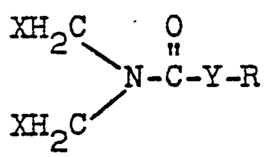

I,

worin R einen aliphatischen, cycloaliphatischen, araliphatischen, aliphatischaromatischen, aromatischen oder heterocyclischen Rest bedeutet, Y ein Schwefelatom bezeichnet, darüber hinaus Y auch für ein Sauerstoffatom, wenn R einen aromatischen oder heterocyclischen Rest bedeutet, stehen kann, die einzelnen Reste X gleich oder verschieden sein können und jeweils für ein Chloratom, Bromatom oder Jodatom stehen, vorteilhaft erhält, wenn man Bis-(N-Halogenmethyl)-carbamidsäurehalogenide der Formel

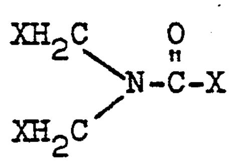

II,

worin die einzelnen Reste X gleich oder verschieden sein

können und jeweils die vorgenannte Bedeutung besitzen, mit Mercaptanen oder Hydroxylverbindungen der Formel

H-Y-R III,

worin Y und R die vorgenannte Bedeutung besitzen, und basischen Verbindungen oder Salzen der Mercaptane oder Hydroxylverbindungen III umsetzt.

Weiterhin wurden die neuen Bis-(N-Halogenmethyl)-carbamidsäureester der Formel

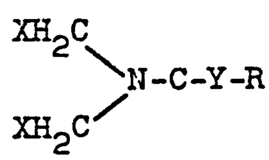 I,

worin R einen aliphatischen, cycloaliphatischen, araliphatischen, aliphatischaromatischen, aromatischen oder heterocyclischen Rest bedeutet, Y ein Schwefelatom bezeichnet, darüber hinaus Y auch für ein Sauerstoffatom, wenn R einen aromatischen oder heterocyclischen Rest bedeutet, stehen kann, die einzelnen Reste X gleich oder verschieden sein können und jeweils für ein Chloratom, Bromatom oder Jodatom stehen, gefunden.

Die Umsetzung kann für den Fall der Verwendung von Bis-(N-Chlormethyl)-carbamidsäurechlorid und 4-Nitrophenol durch folgende Formeln wiedergegeben werden:

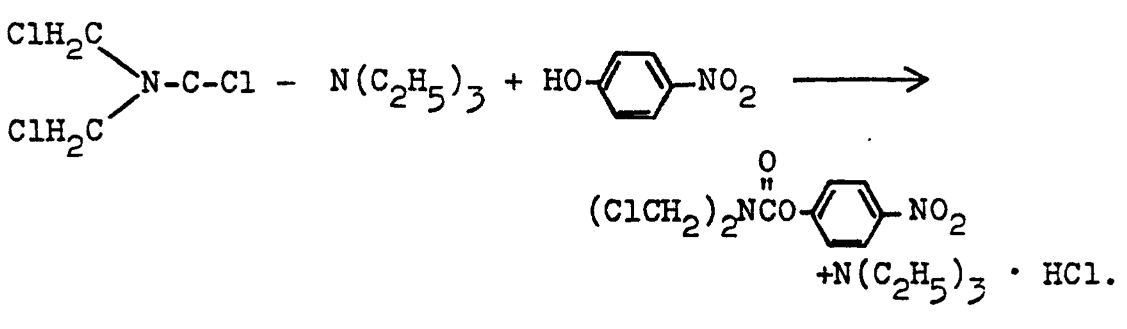

Im Hinblick auf den Stand der Technik liefert das Verfahren nach der Erfindung auf einfachem und wirtschaftlichem Wege die neuen Bis-(N-Halogenmethyl)-carbamidsäureester in guter Ausbeute und Reinheit. Diese vorteilhaften Ergebnisse sind im Hinblick auf die belgische Patentschrift 660 727 überraschend.

Die als Ausgangsstoffe II verwendeten Bis-(N-Halogenmethyl)-carbamidsäurehalogenide können z.B. nach den in der deutschen Auslegeschrift 11 54 087 und der deutschen Patentschrift 11 32 118 beschriebenen Verfahren durch Halogenierung von Bismethylcarbamidsäurehalogeniden bzw. durch Umsetzung von Hexamethylentetramin mit Phosgen hergestellt werden. Die Ausgangsstoffe II und III können in stöchiometrischer Menge oder im Überschuß, vorzugsweise in einem Verhältnis von 0,9 bis 1,3 Mol Ausgangsstoff III je Mol Ausgangsstoff II umgesetzt. Bevorzugte Ausgangsstoffe II und III und dementsprechend bevorzugte Endstoffe I sind solche, in deren Formeln R jeweils einen Alkylrest mit 1 bis 8 Kohlenstoffatomen, einen Alkenylrest mit 3 bis 8 Kohlenstoffatomen, einen Cycloalkylrest mit 4 bis 8 Kohlenstoffatomen, einen Aralkylrest oder Alkylarylrest mit 7 bis 12 Kohlenstoffatomen, einen Phenylrest, einen Naphthylrest, einen 5- bis 6-gliedrigen, heterocyclischen Rest, der neben dem Stickstoffatom noch ein weiteres Stickstoffatom oder ein Sauerstoffatom enthalten kann, bedeutet, Y ein Schwefelatom bezeichnet, darüber hinaus Y auch für ein Sauerstoffatom, wenn R einen aromatischen oder heterocyclischen Rest bedeutet, stehen kann, die einzelnen Reste X gleich oder verschieden sein können und jeweils für ein Jodatom, vorzugsweise Bromatom und insbesondere ein Chloratom stehen. Die vorgenannten Reste und Ringe können noch durch unter den Reaktionsbedingungen inerte Gruppen und/oder Atome, z.B. Chloratome, Alkyl-, Alkoxygruppen mit jeweils 1 bis 4 Kohlenstoffatomen, Nitrogruppen, substituiert sein.

So sind z.B. die folgenden Ausgangsstoffe II geeignet: Bis-[N-Jodmethyl]-carbamidsäurejodid, Bis-[N-Brommethyl]-carbamidsäurebromid, Bis-[N-Chlormethyl]-carbamidsäurechlorid, Bis-[N-Brommethyl]-carbamidsäurechlorid.

Als Ausgangsstoffe III kommen infrage: Phenol, 2-Methylphenol, 3-Methylphenol, 4-Methylphenol, 2-Methoxyphenol, 3-Methoxyphenol, 4-Methoxyphenol, 2,3-Dimethylphenol, 3,4-Dimethylphenol, 2,6-Dimethylphenol, 3,5-Dimethylphenol, 2,3-Dimethoxyphenol, 3,4-Dimethoxyphenol, 3,5-Dimethoxyphenol, 2-Äthylphenol, 3-Äthylphenol, 4-Äthylphenol, 2,3-Diäthylphenol, 3,4-Diäthylphenol, 2,6-Diäthylphenol, 3,5-Diäthylphenol, 2-Äthoxyphenol, 3-Äthoxyphenol, 4-Äthoxyphenol, 2-n-Propylphenol, 3-n-Propylphenol, 4-n-Propylphenol, 2,3-Di-n-propylphenol, 3,4-Di-n-propylphenol, 2,6-Di-n-propylphenol, 3,5-Di-n-propylphenol, 2-Isopropylphenol, 3-Isopropylphenol, 4-Isopropylphenol, 2-Butylphenol, 3-Butylphenol, 4-Butylphenol, 2-Isobutylphenol, 3-Isobutylphenol, 4-Isobutylphenol, 2-tert.-Butylphenol, 3-tert.-Butylphenol, 4-tert.-Butylphenol, 2,3-Diäthoxyphenol, 3,4-Diäthoxyphenol, 2,6-Diäthoxyphenol, 3,5-Diäthoxyphenol, 2-Nitrophenol, 3-Nitrophenol, 4-Nitrophenol, 2-Chlorphenol, 3-Chlorphenol, 4-Chlorphenol, 2,3-, 2,4-, 2,5-, 2,6-Dichlorphenol, 2,4,6-, 2,3,4-, 2,3,5-, 2,3,6-, 2,4,5-Trichlorphenol, 2,3,4,5-, 2,3,4,6-, 2,3,5,6-Tetrachlorphenol, Pentachlorphenol; mit vorgenannten, ganz oder teilweise unterschiedlichen Substituenten zweifach, dreifach, vierfach oder fünffach substituierte Phenole; Naphthole und analog substituierte Naphthole; analoge Alkohole des Imidazols, 1-Methylimidazols, 1-Propylimidazols, 2-Methylpyridins, 3-Methylpyridins, 4-Methylpyridins, 2,4-Dimethylpyridins, 2,6-Dimethylpyridins, 2,4,6-Trimethylpyridins, Pyridins, Pyrrols, Imidazolidins, Piperidins, Morpholins, Pyridazins, Pyrimidins, Pyrazins, Piperazins; analoge Thiole; Methyl-,

Äthyl-, Propyl-, Isopropyl-, n-Butyl-, Isobutyl-, sek.-Butyl-, tert.-Butyl-, Allyl-, Benzyl-, Cyclohexyl-, Cyclopentyl-mercaptan.

Ebenfalls kommen als Ausgangsstoffe die Salze der Mercaptane oder Hydroxylverbindungen III, vorzugsweise die Erdalkali- und insbesondere Alkaliverbindungen, insbesondere die Magnesium-, Lithium-, Kalium- und insbesondere Natriumverbindung, in Betracht.

Die Umsetzung wird im allgemeinen bei einer Temperatur von -30 bis +100, vorzugsweise von 0 bis 60°C, drucklos oder unter Druck, kontinuierlich oder diskontinuierlich durchgeführt. Zweckmäßig verwendet man unter den Reaktionsbedingungen inerte, organische Lösungsmittel. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Nitrile wie Acetonitril, Butyronitril, Isobutyronitril, Benzonitril, m-Chlorbenzonitril; Äther, z.B. Äthylpropyläther, Methyl-tert.-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, Thioanisol; und entsprechende Gemische. Zweckmäßig verwendet man das Lösungsmittel in einer Menge von 400 bis 10 000 Gewichtsprozent, vorzugsweise von 500 bis 4 000 Gewichtsprozent, bezogen auf Ausgangsstoff II.

Die Umsetzung wird in Gegenwart einer basischen Verbindung vorteilhaft in einer Menge von 0,8 bis 1,5, vorzugsweise von 0,9 bis 1,2 Äquivalenten basischer Verbindung, bezogen auf ein Mol Ausgangsstoff II, durchgeführt. Als basische Verbindungen werden zweckmäßig Alkaliverbindungen, Erdalkaliverbindungen, Ammoniumverbindungen und insbesondere tertiäre Amine sowie entsprechende Gemische verwendet.

Bevorzugt sind tertiäre Amine und (Thio)Alkoholate, insbesondere Alkali(thio)alkoholate. Es kommen z.B. als basische Verbindungen in Frage: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Trimethylamin, Triäthylamin, Tripropylamin, Triisopropylamin, Tributylamin, Triisobutylamin, Tri-sek.-butylamin, Tri-tert.-butylamin, Tribenzylamin, Tricyclohexylamin, Triamylamin, Trihexylamin, N,N-Dimethylanilin, N,N-Diäthylanilin, N,N-Dipropylanilin, N,N-Dimethyltoluidin, N,N-Diäthyltoluidin, N,N-Dipropyltoluidin, N,N-Dimethyl-p-aminopyridin, N,N-Diäthyl-p-aminopyridin, N,N-Dipropyl-p-aminopyridin, N,N-Dimethylaminoäthanol, N,N-Diäthylaminoäthanol, N,N-Dipropylaminoäthanol, N-Methylpyrrolidon, N-Äthylpyrrolidon, N-Methylpiperidin, N-Äthylpiperidin, N-Methylpyrrolidin, N-Äthylpyrrolidin, N-Methylimidazol, N-Äthylimidazol, N-Methylpyrrol, N-Äthylpyrrol, N-Methylmorpholin, N-Äthylmorpholin, N-Methylhexamethylenimin, N-Äthylhexamethylenimin, Pyridin, Chinolin, α-Picolin, ß-Picolin, γ-Picolin, Isochinolin, Pyrimidin, Acridin, N,N,N',N'-Tetramethyläthylendiamin, N,N,N',N'-Tetraäthyläthylendiamin, Chinoxalin, Chinazolin, N-Propyldiisopropylamin, N,N-Dimethylcyclohexalamin, 2,6-Lutidin, 2,4-Lutidin, Trifurfurylamin, Triäthylendiamin. Im allgemeinen werden die Mengen der Komponenten so gewählt, daß das Reaktionsgemisch während der Umsetzung einen pH von mindestens 7, vorzugsweise über 7, aufweist.

Die Reaktion kann wie folgt durchgeführt werden: Ein Gemisch der Ausgangsstoffe II und III und basischer Verbindung und vorteilhaft Lösungsmittel wird während 2 bis 15 Stunden bei der Reaktionstemperatur gehalten. Das Carbamidsäurechlorid II wird zweckmäßig im Lösungsmittel vor-

gelegt, der Ausgangsstoff III und anschließend die basische Verbindung unter Rühren zugegeben. Dann wird der Endstoff aus dem Gemisch in üblicher Weise, z.B. durch Kristallisation und Filtration, abgetrennt.

Die nach dem Verfahren der Erfindung hergestellten neuen Bis-(N-Halogenmethyl)-carbamidsäureester I sind wertvolle Ausgangsstoffe für die Herstellung von Farbstoffen, Pflanzenschutzmitteln, Textilausrüstungsmitteln zur Hochveredelung.

Die in den folgenden Beispielen aufgeführten Teile bedeuten Gewichtsteile.

Beispiel 1

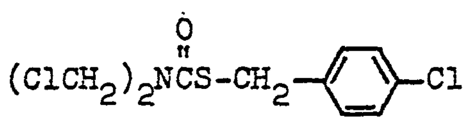

In 100 Teile Toluol werden 17,6 Teile Bis-(N-Chlormethyl)-carbamidsäurechlorid vorgelegt. Man gibt bei 20°C 15,9 Teile para-Chlorbenzylmercaptan und dann langsam unter Rühren 10,1 Teile Triäthylamin zu. Dabei steigt die Temperatur auf 60°C an. Man rührt 2 Stunden bei 20 bis 30°C nach. Der entstandene Niederschlag wird abgesaugt. Man engt das Filtrat ein und filtriert den ausgeschiedenen Endstoff ab. Man erhält 19,5 Teile Bis-(N-Chlormethyl)-carbamidsäure-4-chlorbenzylthiolester (65 % der Theorie) vom Schmelzpunkt 95 bis 97°C (Cyclohexan).

Beispiel 2

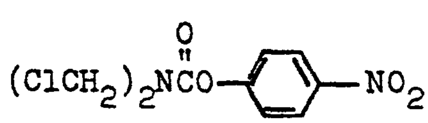

8,83 Teile Bis-(N-Chlormethyl)-carbamidsäurechlorid in 150 Teilen Äther werden auf -20°C abgekühlt. Man gibt 6,95 Teile 4-Nitrophenol und dann bei 0°C 5,05 Teile Triäthylamin zu. Man rührt eine Stunde bei 0°C nach, anschließend bei Raumtemperatur 14 Stunden. Man filtriert den Feststoff ab und engt das Filtrat ein. Man erhält 13 Teile Bis-(N-Chlormethyl)-carbamidsäure-4-nitrophenylester (93 % der Theorie) in Gestalt eines Öles mit dem Brechungsindex $n_D^{26} = 1,5697$.

Beispiel 3

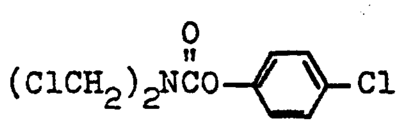

19,42 Teile Bis-(N-Chlormethyl)-carbamidsäurechlorid werden in 150 Teilen Äther vorgelegt. Zu dieser Lösung gibt man unter Feuchtigkeitsausschluß bei -20°C unter Rühren portionsweise das Natriumsalz von 11,75 Teilen 4-Chlorphenol. Man rührt eine Stunde bei 0°C, anschließend 2 Stunden bei 20°C. Man filtriert ab und engt das Filtrat ein. Man erhält 21 Teile Bis-(N-Chlormethyl)-carbamidsäure-4-chlorphenylester (87 % der Theorie) in Gestalt eines farblosen Öles mit dem Brechungsindex $n_D^{31,5} = 1,5513$.

Beispiele 4 bis 21

Analog Beispiel 1 bis 3 werden folgende Verbindungen A hergestellt:

Tabelle

$A = (XCH_2)_2N\overset{O}{\overset{\|}{C}}-YR$

| Beispiel | Mol Ausgangs-stoff II | Mol Ausgangs-stoff III | Mol Base | Base | -R | X | Y | | Ausbeute % der Theorie | Verfahrensweise analog Beispiel |
|---|---|---|---|---|---|---|---|---|---|---|
| 4 | 0,1 | 0,1 | 0,1 | $N(C_2H_5)_3$ | $-C_2H_5$ | Cl | S | Kp 142-146°C/29mbar | 70 | 1 |
| 5 | 0,1 | 0,1 | 0,1 | " | $-CH(CH_3)_2$ | Cl | S | Kp 147-151°C/33mbar | 68 | 1 |
| 6 | 0,1 | 0,1 | 0,1 | " | $-CH_2CH{=}CH_2$ | Cl | S | Kp 166-170°C/47mbar | 85 | 1 |
| 7 | 0,1 | 0,1 | 0,1 | " | $-CH_2-$ 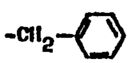 | Cl | S | Kp 139-141°C/ 0,01 mbar | 80 | 1 |
| 8 | 0,05 | 0,05 | 0,05 | " | 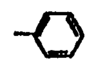 | Cl | O | $n_D^{23}$ = 1,5432 | 85 | 2 |
| 9 | 0,05 | 0,05 | 0,05 | " | 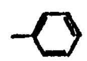 | Cl | S | Fp 68-71°C | 80 | 2 |
| 10 | 0,05 | 0,05 | 0,05 | " | 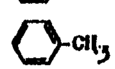 | Cl | O | $n_D^{23}$ = 1,5389 | 78 | 2 |
| 11 | 0,05 | 0,05 | 0,05 | " | 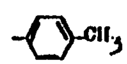 | Cl | S | Fp 84-86°C | 70 | 1 |
| 12 | 0,05 | 0,05 | 0,05 | " | 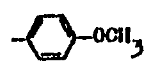 | Cl | O | $n_D^{26}$ = 1,5462 | 76 | 2 |
| 13 | 0,1 | 0,1 | 0,1 | " | 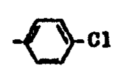 | Cl | S | Kp 154-156°C/ 0,13 mbar | 80 | 1 |
| 14 | 0,1 | 0,1 | 0,1 | " | 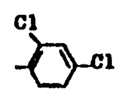 | Cl | O | Fp 44-48°C | 50 | 2 |
| 15 | 0,1 | 0,1 | 0,1 | " | 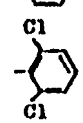 | Cl | O | Fp 93-94°C | 60 | 2 |

Tabelle (Fortsetzung)

$A = (XCH_2)_2N\overset{O}{\overset{\|}{C}}-YR$

| Beispiel | Mol Ausgangsstoff II | Mol Ausgangsstoff III | Mol Base | Base | -R | X | Y | | Ausbeute % der Theorie | Verfahrensweise analog Beispiel |
|---|---|---|---|---|---|---|---|---|---|---|
| 16 | 0,1 | 0,1 | 0,1 | $N(C_2H_5)_3$ | 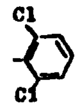 | Cl | S | Fp 115-117°C | 85 | 1 |
| 17 | 0,08 | 0,08 | 0,08 | " | 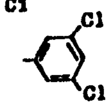 | Cl | O | Kernresonanzsignal bei $\delta$ = 5,32 ppm (in $CDCl_3$, bezogen auf Tetramethylsilan) | 60 | 2 |
| 18 | 0,1 | 0,1 | 0,1 | " | 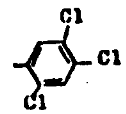 | Cl | S | Fp 96-98°C | 50 | 1 |
| 19 | 0,1 | 0,1 | 0,1 | " | 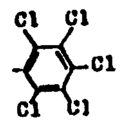 | Cl | O | Fp 156-158°C | 73 | 2 |
| 20 | 0,1 | 0,1 | 0,1 | " | " | Cl | S | Fp 154-156°C | 85 | 1 |
| 21 | 0,07 | 0,07 | 0,07 | " | 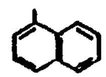 | Cl | O | Fp 84-88°C | 30 | 2 |

## Patentansprüche

1. Verfahren zur Herstellung von Bis-(N-Halogenmethyl)-carbamidsäureester der Formel

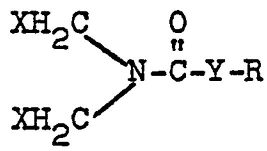 I,

worin R einen aliphatischen, cycloaliphatischen, araliphatischen, aliphatischaromatischen, aromatischen oder heterocyclischen Rest bedeutet, Y ein Schwefelatom bezeichnet, darüber hinaus Y auch für ein Sauerstoffatom, wenn R einen aromatischen oder heterocyclischen Rest bedeutet, stehen kann, die einzelnen Reste X gleich oder verschieden sein können und jeweils für ein Chloratom, Bromatom oder Jodatom stehen, <u>dadurch gekennzeichnet</u>, daß man Bis-(N-halogenmethyl)-carbamidsäurehalogenide der Formel

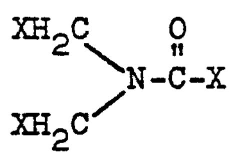 II,

worin die einzelnen Reste X gleich oder verschieden sein können und jeweils die vorgenannte Bedeutung besitzen, mit Mercaptanen oder Hydroxylverbindungen der Formel

H-Y-R III,

worin Y und R die vorgenannte Bedeutung besitzen, und basischen Verbindungen oder Salzen der Mercaptane oder Hydroxylverbindungen III umsetzt.

2. Bis-(N-Halogenmethyl)-carbamidsäureester der Formel

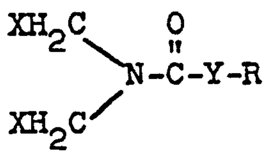 I,

worin R einen aliphatischen, cycloaliphatischen, araliphatischen, aliphatischaromatischen, aromatischen oder heterocyclischen Rest bedeutet, Y ein Schwefelatom bezeichnet, darüber hinaus Y auch für ein Sauerstoffatom, wenn R einen aromatischen oder heterocyclischen Rest bedeutet, stehen kann, die einzelnen Reste X gleich oder verschieden sein können und jeweils für ein Chloratom, Bromatom oder Jodatom stehen.

0006990

Europäisches Patentamt

# EUROPÄISCHER RECHERCHENBERICHT

Nummer der Anmeldung
79101618

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| D | DE-A-1 768 983 (HOECHST) *Beispiele 1-18* | 1 | C07C 155/02<br>C07C 125/06 |
| | BE-A-633 027 (BAYER) * Beispiel 22a* | 1 | |
| | BE-A-621 373 (BAYER) *Beispiele 8-10,14-16,18,19* | 1 | |
| D | BE-A-660 727 (BASF) *Beispiel 2,3* | 1 | RECHERCHIERTE SACHGEBIETE (Int. Cl.²)<br>C07C 155/00,02<br>C07C 125/00,05 |
| D | DE-B-1 154 027 (BASF) *Beispiel 1,6* | 1 | |
| D | DE-B- 1 132 118 (BAYER) *Beispiele 1,2* | 1 | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Wien | 25.Juli 1979 | Daschl |

EPA form 1503.1 06.78